# EUROPEAN PATENT APPLICATION

(11) **EP 3 750 546 A1**
(43) Date of publication of application: **16.12.2020**
(21) Application number: 19750500.1
(22) Date of filing: 08.02.2019
(51) Int. Cl.: A61K 35/10, A23L 33/10, A61P 3/10, A61K 36/15

(54) **GLYCEROL RELEASE PROMOTER**

(30) Priority: 09.02.2018 JP 2018035789
(71) Applicant: Kohaku Bio Technology Co., Ltd., Ibaraki 3050006 (JP)
(72) Inventor: SAKAMOTO Kazuichi, Tsukuba-shi Ibaraki 305-8577 (JP); SOGO Erika, Tsukuba-shi Ibaraki 305-8577 (JP); TAKEDA Reiko, Tsukuba-shi Ibaraki 305-0006 (JP); HAEIWA Haruna, Tsukuba-shi Ibaraki 305-0006 (JP); YAMAMOTO Takuya, Tsukuba-shi Ibaraki 305-0006 (JP); YAMANO Mikio, Tsukuba-shi Ibaraki 305-0006 (JP)
(74) Representative: Schön, Christoph
(86) International application number: PCT/JP2019/004599
(87) International publication number: WO 2019/156213

(57) **Abstract**

Problem to be solved

To prevent and improve obesity on a long-term basis by promoting the extracellular release of glycerol formed by lipolysis in adipose tissues and internal organs.

Solution

A glycerol release promoter including an amber extract as an active ingredient.

## Description

### Technical Field

The present invention relates to a glycerol release promoter containing an amber extract.

### Background Art

Amber is a fossil formed mainly by the burial and condensation of the resin of Pinus plants underground for a long period of time, and its powder has been used as a Chinese medicine since ancient times in China. It mainly contains resin, essential oils, succinic acid, and the like, and dissolves in small amounts in ethanol, diethyl ether, or benzene (for example, see Non Patent Literature 1).

It is well known as jewelry in Japan, but in recent years, its powder and extract is increasingly used in cosmetics and health foods. For example, a technique of incorporating amber powder in a cosmetic to improve the skin texture (see, for example, Patent Literature 1), a technique of incorporating an amber extract in an external skin preparation (see, for example, Patent Literature 2 and Patent Literature 3), a technique utilizing the whitening effect of an amber extract (see, for example, Patent Literature 4 and Patent Literature 5), a technique utilizing a skin turnover promoting factor in an amber extract fraction (see, for example, Patent Literature 6), a technique utilizing the skin firming effect of an amber extract (see, for example, Patent Literature 7), a technique utilizing a hyaluronic acid production promoting factor in an amber extract fraction (see, for example, Patent Literature 8), and a technique utilizing an angiogenesis promoting factor in an amber extract fraction (see, for example, Patent Literature 9) are known.

In this way, amber is known for various physicochemical and biological effects, has been used in various fields as a very useful material, and is showing promise as a material having infinite potential.

Obesity refers to a condition in which the intake energy exceeds the consumed energy, causing the excess to accumulate as fat in the subcutaneous tissues and internal organs, which results in weight gain and a fat appearance. Together with lifestyle-related diseases, it is called metabolic syndrome and has become a social problem as a risk factor causing the three major diseases, cancer, brain disease, and heart disease.

There are various methods to eliminate obesity, such as diet control, sports, exercise, and drug therapy, but in the modern society, which is full of delicious foods and various entertainments, there are many people who wish to stay healthy and not suffer from any lifestyle-related disease, without lowering such quality of life. That is, a method which does not allow fat to accumulate while eating what one wants is the most desired form.

Lipase, an enzyme that decomposes fat into fatty acids and glycerol, is known as a method for decomposing fat accumulated in the subcutaneous tissues or the internal organs, and the decomposition of fat progresses further when lipases are activated. However, if none of the decomposition products are released outside the adipose tissues or internal organs, the enzymatic reaction will eventually be saturated and the effect will be weakened. Therefore, it is important to activate the releasing action of either the fatty acids or glycerol.

Paeoniflorin, which promotes the release of glycerol into the culture medium of cultured adipocytes, is known to exhibit such action (see, for example, Patent Literature 10).

### Citation List

### Patent Literature

Patent Literature 1: Japanese Patent Laid-Open No. 2004-83478
Patent Literature 2: Japanese Patent Laid-Open No. H9-227334
Patent Literature 3: Japanese Patent Laid-Open No. 2001-131048
Patent Literature 4: Japanese Patent Laid-Open No. 2010-235551
Patent Literature 5: Japanese Patent Laid-Open No. 2012-240967
Patent Literature 6: Japanese Patent Laid-Open No. 2007-314522
Patent Literature 7: Japanese Patent Laid-Open No. 2008-189669
Patent Literature 8: Japanese Patent Laid-Open No. 2008-266260
Patent Literature 9: Japanese Patent Laid-Open No. 2011-256164
Patent Literature 10: National Publication of International Patent Application No. 2011-523950

### Non Patent Literature

Non Patent Literature 1: The Great Dictionary of Chinese Medicinals, Volume 2, Shanghai Science and Technology Publishing Co. (Edited by Jiangsu New Medical College, "The Great Dictionary of Chinese Medicinals", Shogakukan) see publicly known literature 1

### Summary of Invention

### Technical Problem

An object of the present invention is to provide a glycerol release promoter for promoting lipolysis, that can be incorporated into foods and drinks which are easy to take on a daily basis.

### Solution to Problem

In view of such circumstances, the present inventors have conducted intensive studies, and as a result, have found an effect of promoting glycerol release in adipocytes or internal organs for an amber extract, and completed the present invention. That is, the present invention is as shown below.
<1> A glycerol release promoter comprising an amber extract.
<2> A lipolysis promoter comprising the glycerol release promoter according to <1>.
<3> The promoter according to <1> or <2>, wherein an extraction solvent for producing the amber extract is a hydrous alcohol (water content: 1 mass% to 60 mass%).
<4> A composition for oral administration, formulated from the promoter according to any one of <1> to <3>.
<5> The composition for oral administration according to <4>, wherein the composition is in the form of a drug, a quasi drug, a food, a drink, or a food additive.

### Advantageous Effects of Invention

The glycerol release promoter containing an amber extract of the present invention can promote lipolysis in adipocytes or internal organs by promoting the extracellular release of glycerol produced by the decomposition of fat accumulated in adipocytes or internal organs. Therefore, adding these promoters to a food or drink allows to efficiently eliminate fat derived from meals even when ingested, which allows to provide a food or drink useful for long-term prevention and improvement of obesity.

### Brief Description of Drawing

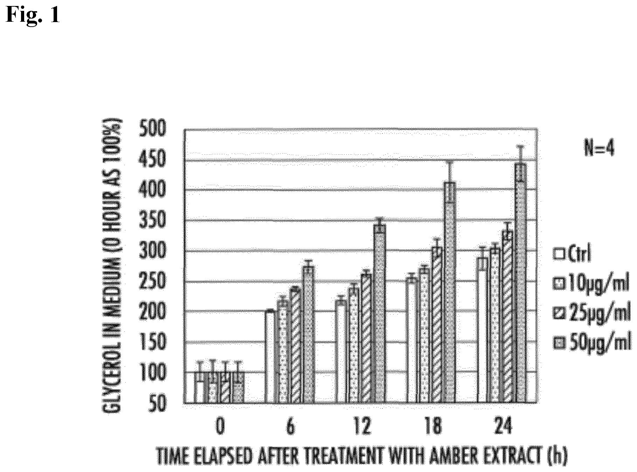

[Figure 1] A graph showing glycerol release promotion by the amber extract of Production Example 1 in 3T3-L1 differentiated cells

### Description of Embodiments

### <Amber extract of the present invention>

The glycerol release promoter of the present invention contains an amber extract as an active ingredient. Here, examples of the amber extract referred to in the present invention include amber itself, a processed product such as crushed or finely cut amber, an extract obtained by adding a solvent to amber or its processed product, a product obtained by removing the solvent from the extract, and the purified products thereof, among which an extract or a product obtained by removing the solvent thereof is particularly preferable. Moreover, the production area of the amber used in the present invention is not particularly limited, but considering the production amount and reserve, a preferable example is amber produced in Kaliningrad, Russia.

Examples of the solvent of the extract include water, alcohols such as methanol or ethanol, 1,3-butanediol, propylene glycol and glycerin, esters such as ethyl acetate and methyl formate, nitriles such as acetonitrile, ethers such as diethyl ether and tetrahydrofuran, halogenated hydrocarbons such as chloroform and methylene chloride, and ketones such as acetone and methyl ethyl ketone, and one or two or more of these may be used alone or in combination. Among these, water or alcohols are preferable, and hydrous alcohol is more preferable. Furthermore, ethanol is preferable as the alcohol. The water content is preferably 1 mass% to 60 mass%, further preferably 10 mass% to 50 mass%. If it is higher or lower than this, the extraction efficiency may be reduced.

An example of extraction method may be adding 2 to 20 times amount of solvent to crushed amber and immerse it for several days, if at room temperature, and for several hours, if at a temperature near the boiling point. Then, the insoluble matter may be removed by filtration or the like, and concentrated under reduced pressure or the like. Moreover, this may be purified by column chromatography using a column packed with silica gel, octadecyl-silylated silica gel, ion exchange resin and the like.

### <Production Example 1>

100 g of amber powder from Kaliningrad, Russia was extracted with 50% ethanol, which was then concentrated under reduced pressure and freeze-dried to obtain 10 g of 50% ethanol extract.

As a method for investigating the glycerol release promoting effect using the obtained extract, for example, a method, in which the differentiation of 3T3-L1 mouse fibroblasts (hereinafter referred to as 3T3-L1 cells) into adipocytes is induced, then the extract is added and cultured, and the glycerol released in the culture medium is quantified, can be used.

Examples of drinks containing the extract of the present invention include tea drinks, coffee drinks, soft drinks, liquor, milk drinks, carbonated drinks, healthy drinks, energy drinks, and sports drinks, and the concentrated liquids and prepared powders of these drinks. Examples of foods include gums, candies, jellies, tablet candies, health foods, nutraceuticals, and supplements.

When the extract of the present invention is used as a medicine such as a prophylactic against obesity, it is provided in the form of powder, granules, tablets, capsules, liquids, injections and the like. The extract of the present invention can be orally administered as it is or diluted with water or the like. Alternatively, the extract is prepared by formulating it with a known pharmaceutical carrier. For example, the extract of the present invention can be administered as an oral liquid preparation such as a syrup, or as an oral solid preparation such as tablets, capsules, granules and powders by processing it into an extract, powder or the like, and blending it with a pharmaceutically acceptable carrier. As the pharmaceutically acceptable carrier, various organic or inorganic carrier substances conventionally used as formulation materials are used, and are blended as excipients, lubricants, binders, or disintegrants in solid formulations, or as solvents, excipients, suspending agents, or binders in liquid formulations. In addition, formulation additives such as preservatives, antioxidants, colorants and sweeteners can be used as necessary.

The food/drink or pharmaceutical composition containing the extract of the present invention can contain the extract of the present invention at any concentration. Preferably, the extract of the present invention is contained in a concentration of 0.01 to 50 mass%, more preferably in a concentration of 0.05 to 20 mass%, with respect to the total amount of the food/drink or the pharmaceutical composition.

In addition, the effective dose can be appropriately determined according to the patient's age and weight, the type and severity of the disease, and the route of administration.

### Example

Hereafter, the present invention will be described in detail using an example, but the present invention is not limited to this example.

### <Example 1> Glycerol release test

3T3-L1 cells were suspended in a culture medium (hereinafter referred to as DMEM medium) in which 10% bovine serum and 1% antibacterial agent (Penicillin-Streptomycin-L-Glutamine) (manufactured by Wako) were added to Dulbecco's Modified Eagle Medium (DMEM) (manufactured by Thermo Fisher Scientific), then were seeded in a 24-well plate. The cells were cultured at 37°C in 95% air-5% carbon dioxide gas and cultured until reaching confluency. After reaching confluency, differentiation was induced with DMI (2.5 µM dexamethasone, 0.5 µM 3-isobutyl-1-methylxanthine and 1.7 µM insulin, manufactured by Sigma). After the differentiation was completed, the culture supernatant was removed by suction, then the amber extract of Production Example 1 (50% ethanol extract) was dissolved in dimethyl sulfoxide (hereinafter referred to as DMSO) so that the final concentrations were 10, 25, and 50 µg/ml, and 500 µl each of the mixtures (DMSO final concentration: 0.25%) were added to the DMEM medium. 35 µl of the culture supernatant was collected at regular time intervals and used as a sample for glycerol quantification. LabAssay™ Triglyceride (manufactured by Wako) reagent was added to a 96-well plate in an amount of 200 µl per well, 20 µl of each of the collected culture supernatants was added to each well, incubation was performed for 10 to 15 minutes, and the absorbance was measured at a wavelength of 600 nm to quantify the glycerol in the culture supernatant.

From the results of Figure 1, it was confirmed that the amber extract of the present invention promotes the release of glycerol in a concentration-dependent manner.

## Claims

1. A glycerol release promoter comprising an amber extract.

2. A lipolysis promoter comprising the glycerol release promoter according to claim 1.

3. The promoter according to claim 1 or 2, wherein an extraction solvent for producing the amber extract is a hydrous alcohol (water content: 1 mass% to 60 mass%).

4. A composition for oral administration, formulated from the promoter according to any one of claims 1 to 3.

5. The composition for oral administration according to claim 4, wherein the composition is in a form of a drug, a quasi drug, a food, a drink, or a food additive.
